# EUROPEAN PATENT APPLICATION

(11) **EP 4 563 175 A1**
(43) Date of publication of application: **04.06.2025**
(21) Application number: 23213030.2
(22) Date of filing: 29.11.2023
(51) Int. Cl.: A61M 5/32

(54) **NEEDLE SAFETY DEVICE FOR A MEDICAL DEVICE**

(71) Applicant: Sensile Medical AG, 4600 Olten (CH)
(72) Inventor: PFRANG, Jürgen, 93183 Kallmünz (DE)
(74) Representative: Bardehle Pagenberg Partnerschaft mbB Patentanwälte Rechtsanwälte

(57) **Abstract**

The present disclosure relates to a needle safety device (1) for a medical device, in particular for a medical injection device (100), the needle safety device (1) comprising: a hub portion (10) defining a longitudinal axis (11), wherein the hub portion (10) comprises a cannula (12) extending from the hub portion (10) substantially along the longitudinal axis (11) and a carrier element (60) arranged on the cannula (12), wherein the cannula (12) and the carrier element (60) are configured to be moved together along the longitudinal axis (11) relative to the hub portion (10); a tubular arrangement (20) being movably arranged on the hub portion (10) to move along the longitudinal axis (11) relative to the hub portion (10), wherein the tubular arrangement (20) is configured to be moved between at least an initial position (24) and an injection position (25), wherein in the injection position, a proximal end (12b) of the cannula (12) is configured to protrude through a septum (50) of the medical device such that the cannula (12) is in fluid communication with a liquid reservoir of the medical device, wherein the tubular arrangement (20) comprises coupling means (35), configured to engage with the carrier element (60) such that the proximal end (12b) of the cannula (12) is configured to be moved out of the septum (50) when the tubular arrangement (20) is moved from the injection position (25) to the initial position (24).

## Description

### 1. Technical field

The present disclosure relates to a needle safety device for a medical device, in particular a medical injection device and a medical device comprising the needle safety device.

### 2. Prior art

Medical devices, in particular medical injection devices, such as auto-injectors or injection pens, are known and popular among users as they provide various benefits and can be used for various applications. An auto-injector is a device with a pre-filled syringe or vial containing a medication. It is typically designed to be automated, meaning that a user may not need to manually load the medication or set the dose. Autoinjectors are often designed to be more user-friendly and may have features like automatic needle insertion and medication delivery at the push of a button. An injection pen is a device that typically comprises a cartridge or a vial containing the medication and a pen-like device with a needle at the tip. It usually requires manual assembly of the cartridge or vial into the pen and the setting of the desired dose before injection. The user typically needs to push a button or plunger to release the medication.

Auto-injectors are usually designed for single-purpose usage. Examples may be an auto-injector that injects an emergency medication like adrenaline in the event of an allergic shock. In such a case, exposure to a microbiological contamination after usage may not be harmful to the user, as the injection is substantially finished. Further, auto-injector may be regularly operated by non-medical personnel. Injection pens having a cartridge (or generally speaking any kind of liquid reservoir comprising a medication) can be designed to be single-patient pens. The user is typically instructed to change the needle for each injection. If the user does not change the needle after each injection, the sealing of the cartridge remains in a pierced status by virtue of the needle. The sealing may be a septum, which serves the purpose to prevent the medication from becoming contaminated. Since the sealing remains in such a pierced status until the next injection, this could lead to contamination. Such disadvantages may be particularly pronounced when the medical injection device is less frequently used for injections.

The potential risk described above that the medication could become contaminated applies to any kind of medical device, in particular medical injection devices, such as the ones being operated by non-medical personnel, e.g., auto-injectors or injection pens. For instance, when the medical injection device has been used on an injection area, it should be ensured that a sealing to the cartridge is provided. This bears the potential to prevent contamination of the medication. For instance, growth of microorganisms through the needle into the liquid reservoir may be prevented. In addition, it should be ensured that the needle should not be contacted by the user when the medical device has been used, so as to prevent any contamination of the user and of the needle.

Needle safety devices have been proposed in the past to address some of the above needs. As background to the present application, the following documents may be mentioned. Unpublished EP applications No. 23154701.9 and No. 23154698.7 both relate to a needle safety device.

However, the proposed solutions still do not lead to optimum results. Hence, improvements are called for.

Thus, it is an object of the present disclosure to provide a needle safety device for a medical device, in particular for a medical injection device, that addresses the aforementioned needs and that overcomes the disadvantages of the present solutions at least partially.

### 3. Summary of the invention

The above-mentioned object is at least partially achieved by the subject-matter of the independent claims. Preferred embodiments are subject of the dependent claims, and other suitable aspects of the present invention are described through the overall disclosure of the present application.

The object is achieved by a needle safety device for a medical device, in particular for a medical injection device, the needle safety device comprising: a hub portion defining a longitudinal axis, wherein the hub portion comprises a cannula extending from the hub portion substantially along the longitudinal axis and a carrier element arranged on the cannula, wherein the cannula and the carrier element are configured to be moved together along the longitudinal axis relative to the hub portion; a tubular arrangement being movably arranged on the hub portion to move along the longitudinal axis relative to the hub portion, wherein the tubular arrangement is configured to be moved between at least an initial position and an injection position, wherein in the injection position, a proximal end of the cannula is configured to protrude through a septum of the medical device such that the cannula is in fluid communication with a liquid reservoir of the medical device, wherein the tubular arrangement comprises coupling means, configured to engage with the carrier element such that the proximal end of the cannula is configured to be moved out of the septum when the tubular arrangement is moved from the injection position to the initial position.

In this manner, the present disclosure provides an improved needle safety device compared to known needle safety devices. In particular, the needle safety device of the present disclosure facilitates that contamination of a liquid reservoir comprising a medication can be mitigated or reduced. In some cases, such contamination can be fully prevented. Thereby, a user may not need to worry about whether or not a piercing means, such as the cannula, has been removed from the septum and / or from the liquid reservoir comprising a medication. This is, as the needle safety device according to the present disclosure is configured to move out the proximal end of the cannula substantially automatically, when a pressure on a contact surface of an injection area is released. In this manner, a substantially fail-safe and more secure as well as sterile medical device can be provided to the user. This makes usage of the needle safety device and in particular of the medical device comprising the needle safety device more convenient, comfortable and less cumbersome to the user. In particular, a user does not have to remove the cannula manually after each usage of the medical device. The needle device may be fixedly arranged with a housing of the medical device. In some examples, said fixing may be substantially permanently. However, it is also encompassed in the present disclosure that the needle safety device is releasably fixed to said housing of the medical device. In this manner, user comfort and the applicability of the needle safety device and / or the medical device can be increased.

The "hub portion" as used in the present disclosure may be an elongated hub portion. In some examples, the longitudinal axis of the hub portion may also be a longitudinal center axis of the hub portion. This may particularly be the case, if the hub portion has a substantially cylindrical shape. It is understood that manufacturing tolerances may need to be considered. Thus, even when the hub portion is said to have a cylindrical shape, the shape of the hub portion may slightly deviate from a cylindrical shape. In some examples, a cross section of the hub portion may have a substantially annular shape. The hub portion may be configured such that when the needle safety device is mounted to a medical device, it may not be able to be rotated and / or moved relatively to the medical device.

The "cannula" as used in the present disclosure may additionally or alternatively be referred to as a needle or injection needle. The cannula may be hollow, so as to guide a fluid therethrough. Further, the cannula may be attached directly or indirectly to the hub portion. The cannula may be part of a cannula arrangement. For instance, the cannula arrangement may comprise a plurality of cannulas. The plurality of cannulas may be connected to one another, for instance along one direction, in particular along the longitudinal axis of the hub portion. The plurality of cannulas may partially overlap one another along the longitudinal axis, which may serve the purpose to fix the cannula arrangement.

The "carrier element" as used in the present disclosure may be understood as an element that is arranged so as to carry the cannula. The term to "carry" merely means that the carrier element should be arranged such that it can move the cannula at least partially by way of its own motion. This may be realized for instance by attaching the cannula and the carrier element to one another. Any kind of suitable attachment means are encompassed in the present disclosure. Such attaching and variations thereof includes the joining of the cannula and the carrier element directly or indirectly to one another. The joining may be stationary (e.g., permanent, or fixed) or moveable (e.g., removable or releasable). The joining may be achieved with the two parts (cannula and carrier element) coupled directly to each other, with the two parts coupled with each other using a separate intervening part and / or any additional intermediate parts coupled with one another, or with the two parts coupled with each other using an intervening part that is integrally formed as a single unitary body with one of the two parts. The attaching may comprise any means of mechanical attachment. Further, the attachment may include a form fit connection of the two parts. This may create a tight and secure fit without the need for additional fasteners or adhesives. Thereby, the attachment may rely on the geometry of the cannula and the carrier element to create a stable and reliable attachment. However, usage of additional means for attachment is not precluded thereby. In one example, the cannula and the carrier element may be permanently or at least for a certain period of time fixed to one another, i.e., such that a relative position of the parts does not substantially vary.

The cannula and the carrier element may be designed to be movable. In particular, they are configured to be "moved together along the longitudinal axis" relative to the hub portion. In this manner, the cannula may be able to realize a penetration of a liquid reservoir within the medical device as described elsewhere herein in greater detail. It is noted that the cannular and the carrier element may additionally or alternative also be moved together along a different axis not parallel to the longitudinal axis. This may depend on the usage of the needle safety device. However, during ordinary use of the needle safety device, they may exclusively move together along the longitudinal axis. In preferred examples, the cannula and the carrier element may be moved together parallel to the injection direction. In preferred examples, the longitudinal axis may be parallel to the injection direction.

The tubular arrangement may comprise one or more elements as described elsewhere herein. The tubular arrangement and / or the one or more elements comprised by it may be a component or structure having a cylindrical or tube-like shape. As understood, manufacturing tolerances need to be included such that deviations from a cylindrical or tube-like shape need to be considered. In some examples, the tubular arrangement may have a rounded shape. The tubular arrangement and / or the one or more elements comprised by it may define a hollow portion, such as a chamber within their periphery. Such hollow portion may extend completely through the tubular arrangement along the axial direction, so as to accommodate one or more other parts. As described elsewhere herein, the tubular arrangement may comprise a first tubular element and a second tubular element.

It is noted that when one or more parts are described in here to "move along the longitudinal axis", this may in some examples mean that the movement is exclusively along the longitudinal axis. However, in other examples, it does not exclude that a movement along an axis not parallel to the longitudinal axis is additionally possible. As understood, in some cases, such a movement along a different axis may be desirable. This may depend on the specific use case of the needle safety device.

When the tubular arrangement is described to be configured to be moved between at least an "initial position" and an "injection position", this encompasses a plurality of intermediate positions between the initial position and the injection position. In addition, the tubular arrangement may be configured to be moved in an "after-injection" position. In some examples, the after-injection position may correspond to the initial position, however, this is not necessary. The at least initial position and the injection position may correspond to two end positions of the tubular arrangement. Said two end positions may be two end positions along the longitudinal axis. This may mean that beyond each of the two end positions no further movement may be possible. For instance, when the tubular arrangement is in the injection position, there may be no further movement along the longitudinal axis in the proximal direction possible, although in some examples, this is not precluded. Further, when the tubular arrangement is in the initial position, there may be no further movement along the longitudinal axis in the distal direction possible, although in some examples, this is not precluded. As understood, the tubular arrangement may be moved in an after-injection position. In some examples, the after-injection position may be the same as the initial position. However, in some examples, the after-injection position maybe different from the initial position. In one example, when the tubular arrangement is in the after-injection position, the tubular arrangement may be positioned further in the proximal direction compared to the initial position. In one example, when the tubular arrangement is in the after-injection position, the tubular arrangement may be positioned further in the distal direction compared to the initial position.

The "injection position" may be a position in which a medication can be guided through the cannula to an injection area, such as a skin of a user. The injection position may typically mean that injection can take place.

The "initial position" may be a position in which the medication guided through the cannula when the tubular arrangement is in the injection position, may not be guided through the cannula anymore. However, as understood by the skilled person, the cannula may still contain residual components of the medication, such as residual components that may not have found their way to the injection area. Further, the cannula itself may still allow that a medication can be guided therethrough, e.g., by pouring a medication through it. However, as described elsewhere, in the initial position, there may be no fluid communication between the liquid reservoir of the medical device and the cannula anymore.

The "after-injection position" may be (same as with the initial position) a position in which the medication guided through the cannula when the tubular arrangement is in the injection position, may not be guided through the cannula anymore. Whenever the tubular arrangement is in the after-injection position, the tubular arrangement is positioned further in the distal direction compared to the injection position.

In the injection position, a proximal end of the cannula is configured to protrude through a septum of the medical device such that the cannula is in fluid communication with a liquid reservoir of the medical device. The "proximal end" of the cannula may be the end thereof that faces a septum of the medical device, when the needle safety device is ordinarily mounted on the medical device. The term "to protrude" may mean that the septum is pierced or that any means are provided such that a fluid communication through the septum may be allowed.

To be "in fluid communication" means that a fluid, such as gas, liquid, may be guided from a liquid reservoir to the cannula and / or vice versa. It is noted that a direct flow path, i.e., from the liquid reservoir to the cannula is not necessary, although such an arrangement is not precluded and, in some cases, preferred. As an example, in the injection position, the cannula may be in fluid communication with one or more chambers and said one or more chambers may be in fluid communication with the liquid reservoir. It is preferred that the liquid reservoir comprises a medication, such that the medication can be guided through the cannula to the injection area of the user for injection.

It is noted that the needle safety device does not comprise the septum and the liquid reservoir, however, the needle safety device needs to be configured to allow for the proximal end of the cannula to protrude the septum and for it to be moved out of the septum as described elsewhere herein. Thereby, it is understood that the needle safety device needs to show these features, for instance when it is mounted on any medical device. Thereby, the skilled person easily recognizes and acknowledges the features and advances that the needle safety device provides without the need to rely on a specific medical device.

The "coupling means" as used in the present disclosure may be understood as any means that can be coupled with, in particular releasably coupled with the carrier element. In particular, the coupling means is be configured to engage with the carrier element. To be engaged may mean to be in contact with one another, such that a transfer of movement of the coupling means to the carrier element may take place. Such engagement may be a direct or an indirect engagement. For instance, the coupling means and the carrier element may come directly in contact with one another. In other examples, the coupling means and the carrier element may come indirectly in contact with one another. The latter may be achieved by employing one or more parts, in particular intermediate parts, engaging with one or both of the carrier element and the coupling means. The coupling means may be fixedly arranged with the tubular arrangement, such that they move as one unit. Preferably the coupling means is a coupling element. More preferably, the coupling means is a coupling arm.

When the tubular arrangement is moved from the injection position to the initial position, the proximal end of the cannula is moved out of the septum. This does not mean that the cannular is moved out of the septum merely at a time when the tubular arrangement reaches the initial position. As described elsewhere herein, a plurality of positions are encompassed. Thus, it may be the case that the cannular is moved out of the septum at any time on the way from the injection position to the initial position. Thereby, it also understood that when the tubular arrangement is moved from the injection position to the after-injection position, the proximal end of the cannula is moved out of the septum.

Moving the cannula out of the septum may mean that the proximal end of the cannula substantially does not contact the septum anymore. As described elsewhere herein, this has the advantage that a contamination of the medication within the liquid reservoir can be mitigated. In one example, it may be sufficient that, when the tubular arrangement is moved from the injection position to the initial position, the cannula is not in fluid communication with the liquid reservoir of the medical device anymore. Thereby, the desired effect of a reduced risk of contamination can likewise be achieved. In some examples, this may have the further advantage that the needle safety device can be conveniently removed from the medical device and exchanged with another needle safety device. Said other needle safety device may be fixed to a housing of the medical device as described elsewhere herein. This facilitates that the main part of the medical device can be used multiple times whilst merely the needle safety device is exchanged. This reduces costs.

The medical device may be a medical injection device. Any kind of medical injection devices are included in the present disclosure. Exemplary, the medical injection device may be an auto-injector, an injection pen, and/or a syringe. Preferably, the medical injection device is an auto-injector. This may in particular be the case, since auto-injectors are regularly operated by non-medical personnel, e.g., in emergency situations. Thus, for these examples, the described advances of the present disclosure are particularly pronounced. However, also injection pens and other medical injection devices may significantly benefit from the advantages described in here.

The septum may provide for a sealing function. The septum may aid in maintaining the integrity and sterility of the medication within the liquid reservoir. The septum may be provided by any kind of suitable material including but not limited to rubber or an elastomeric material. The septum may be arranged at an end of the liquid reservoir.

The septum may be able to at least partially close the piercing provided by the cannula upon moving the tubular arrangement from the injection position back to the initial position.

It is understood that the described advantages may apply also for the following preferred embodiments.

In a preferred embodiment of the needle safety device of the present disclosure, the coupling means is configured to be displaced relative to the carrier element, preferably in a direction substantially perpendicular to the longitudinal axis, when the tubular arrangement is moved from the initial position to the injection position. This facilitates that the coupling means can be guided around the carrier element when moved from the initial position to the injection position. It may be the case that the coupling means can be displaced in other directions not being substantially perpendicular to the longitudinal axis.

In a preferred embodiment of the needle safety device of the present disclosure, the coupling means is configured to be in sliding engagement with the carrier element when the tubular arrangement is moved from the initial position to the injection position. This has the advantage that a force required for moving the tubular arrangement does not substantially increase compared to the case when the coupling means is not present. For instance, the sliding engagement entail that a lower friction between the coupling means and the carrier element is provided. Still, the engagement as described further above when the tubular arrangement is moved from the injection position to the initial position can be ensured. It is noted that the sliding engagement is different from the engagement described further above.

In a preferred embodiment of the needle safety device of the present disclosure, the coupling means is resilient, such that it provides a restoring force in a direction substantially perpendicular to the longitudinal axis. This facilitates that the coupling means can be at least partially moved back once it is displaced and once the force causing the displacement is reverted. For instance, the coupling means may be configured to snap back. As an example, once the coupling means is located at least partially beyond a distal end of the carrier element in the proximal direction (i.e., when the tubular arrangement is moved from the initial position to the injection position), the coupling means or a part thereof may snap back. This has the advantage that an engagement can be provided when the tubular arrangement is moved (back) from the injection position to the initial position.

In a preferred embodiment of the needle safety device of the present disclosure, the coupling means has an elongated shape, wherein a longitudinal axis of the coupling means is substantially parallel to the longitudinal axis of the hub portion. This has the advantage that the coupling means can be easily integrated in the needle safety device. This may be the case since the cannula is usually also elongated. The term "elongated" means that there may be a dimension along one axis, which may be larger than one and preferably than both dimensions along the remaining axes, the remaining axes being substantially perpendicular to said one axis. It is understood that when dimensions are described herein, manufacturing tolerances usually have to be taken into consideration. Thus, the dimensions described herein may vary slightly.

In a preferred embodiment of the needle safety device of the present disclosure, the coupling means comprises a snap portion configured to engage with the carrier element when moved from the injection position to the initial position and configured to be in sliding engagement with the carrier element when moved from the initial position to the injection position. The snap portion may be arranged at a proximal end of the coupling means, i.e., at an end that is closer to the medical device when the needle safety device is mounted on the medical device. This snap portion facilitates said engagement and said sliding engagement with the carrier element depending on the direction of movement of the tubular arrangement, and thereby, the movement of the coupling means. The snap portion may be a portion that is integrally formed with the coupling means or a portion that is attached to the coupling means. Any suitable means for attachments are encompassed as described elsewhere herein. As described elsewhere herein, a plurality of positions of the tubular arrangement (e.g., at least initial, injection, and after-injection) are encompassed in the present disclosure. Thus, it may be the case that the snap portion is configured to engage with the carrier element when moved out of the septum at any time on the way from the injection position to the initial position. Thereby, it also understood that when the tubular arrangement is moved from the injection position to the after-injection position, the snap portion is configured to engage with the carrier element.

In a preferred embodiment of the needle safety device of the present disclosure, the snap portion has a tapered shape as seen along the longitudinal axis from a distal end of the coupling means towards a proximal end of the coupling means. This has the advantage that the coupling means can easily slide over the carrier element in one direction and that it can engage with the carrier element in the opposite direction. The tapered shape may be a geometric form that substantially gradually narrows or becomes narrower toward one end thereof, in particular toward a proximal end of the snap portion. The tapered shape may correspond to a conical or pyramidal appearance.

In a preferred embodiment of the needle safety device of the present disclosure, the snap portion has an inclined surface configured to slide over the carrier element, in particular over a protrusion of the carrier element, and an abutting surface for engaging with the carrier element, in particular with a protrusion of the carrier element, wherein the inclined surface is arranged in proximity of a proximal end of the coupling means, wherein the abutting surface is arranged further to a distal end of the coupling means with respect to the inclined surface. This further contributes to the advantages of a relatively smooth movement of the coupling means in one direction (e.g., a sliding engagement) and an engagement in the opposite direction. The proximal end of the coupling means may face the medical device when the needle safety device is mounted thereto. The distal end of the coupling means may be substantially opposite to the proximal end. The distal end of the coupling means may be closer to an injection area during ordinary use of the medical device.

In a preferred embodiment of the needle safety device of the present disclosure, the inclined surface defines an angle with respect to the longitudinal axis of the hub portion of at least 10°, preferably at least 20°, more preferably at least 30°, more preferably at least 40°, more preferably at least 45°, and/or of at most 80°, preferably at most 70°, more preferably at most 60°, more preferably at most 50°, most preferably at most 45°. This further contributes to the advantages set out elsewhere herein, e.g., of providing a relatively smooth movement without structural impairment of the coupling means in one direction and an engagement in the opposite direction. The angle should not be too large as this impairs a smooth movement of the coupling means from the initial position to the injection position. Further, the angle should not be too small, as this may unnecessarily lengthen the snap portion, thereby demanding more material. With the angles as specified in this embodiment, an optimal balance can be struck between these different and conflicting requirements.

In a preferred embodiment of the needle safety device of the present disclosure, the abutting surface is arranged substantially perpendicular to the longitudinal axis of the hub portion. This allows for a relatively easy structural engagement of the coupling means and the carrier element. Further, such an abutting surface may be manufactured in a simplified manner.

In a preferred embodiment of the needle safety device of the present disclosure, the carrier element comprises a protrusion for engaging with the coupling means in particular with the snap portion of the coupling means. This further complements the advantages outlined elsewhere.

In a preferred embodiment of the needle safety device of the present disclosure, the protrusion extends in a direction substantially perpendicular to the longitudinal axis. This embodiment in combination with the abutting surface of the snap portion allows for a combined advantageous effect in that the abutting surface and the protrusion can make a substantially flat contact when they engage when being moved from the injection position to the initial position. Thereby, movement of the carrier element by way of a movement of the coupling means can be provided in a more reliable manner.

In a preferred embodiment of the needle safety device of the present disclosure, the needle safety device is further comprising a spring element which biases the tubular arrangement in a distal direction relative to the hub portion. This may have the advantage that the tubular arrangement can automatically be moved by way of the spring from the injection position to the initial position upon release of pressure on a contact surface of the tubular arrangement (e.g., when the contact surface is not in pressure contact with the injection area, such as a skin of a user).

The object of the present disclosure is also achieved by a medical device, in particular a medical injection device, comprising a needle safety device as described in the present disclosure. As understood, since the medical device comprises the needle safety device as described elsewhere herein, it is understood that the features and/or advantages described with reference to the needle safety device may also apply for the medical device and vice versa.

In a preferred embodiment of the medical device of the present disclosure, the medical device is further comprising a housing which engages with the tubular arrangement, in particular with a second tubular element of the tubular arrangement, such that the second tubular element of the tubular arrangement substantially does not rotate relative to the hub portion, wherein optionally the hub portion is affixed to the housing.

The object of the present disclosure is also achieved by a use of a needle safety device as described in the present disclosure in a medical device, in particular in a medical injection device, for moving the cannula out of the septum after injection. The respective advantages described with reference to the needle safety device may also apply for the use of the needle safety device.

### 4. Brief description of the accompanying figures

In the following, the invention will be described in more detail with reference to the following figures:
- **Fig. 1:**: shows a needle safety device according to the present invention in a cross-sectional cut prior to injection.
- **Fig. 2:**: shows the needle safety device in a cross-sectional cut view during injection.
- **Fig. 3:**: shows the needle safety device in a cross-sectional cut view after injection.
- **Fig. 4:**: shows a medical device according to the present disclosure.

### 5. Detailed description of the figures

In the following only some possible embodiments of the invention are described in detail. However, the present invention is not limited to these, and a multitude of other embodiments are applicable without departing from the scope of the invention. The presented embodiments can be modified in a number of ways and combined with each other whenever compatible and certain features may be omitted in so far as they appear dispensable. In particular, the disclosed embodiments may be modified by combining certain features of one embodiment with one or more features of another embodiment.

Throughout the present figures and specification, the same reference numerals refer to the same elements. For the sake of clarity and conciseness, certain aspects of components or steps of certain embodiments are presented without undue detail where such detail would be apparent to those skilled in the art in light of the teachings herein and / or where such detail would obfuscate an understanding of more pertinent aspects of the embodiments.

### Definitions

The "proximal" end as used herein is merely used to illustrate one of two ends of a part. The proximal end of a part may be understood best in combination with a distal end of the same part. The terms proximal and distal are not to be understood limiting by any means. The ends could additionally or alternatively be referred to as first and second end or second and first end, respectively.

The distal end of a part may usually be an end that is further away from a center point of the medical device compared to the proximal end of the part. Further, the distal end of a part may usually be an end that is closer to an injection area compared to the proximal end of the part when the medical device is used according to its ordinary use.

Same applies to the terms "proximal direction" and "distal direction". That is, these terms could additionally or alternatively be referred to as first and second direction or second and first direction. The proximal direction may be a direction from a distal end to a proximal end. The distal direction may be a direction from a proximal end to a distal end.

Unless otherwise stated, the term "substantial" or "substantially" as used in the present context may be understood to a great or significant extent or for the most part or essentially. In particular, manufacturing tolerances are included by this term.

### Description of preferred embodiments

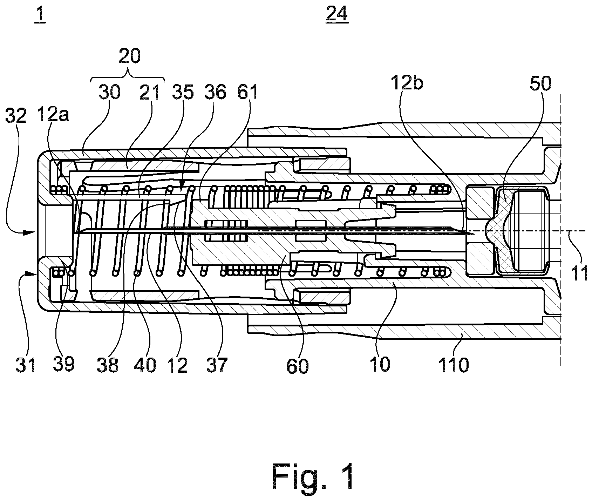

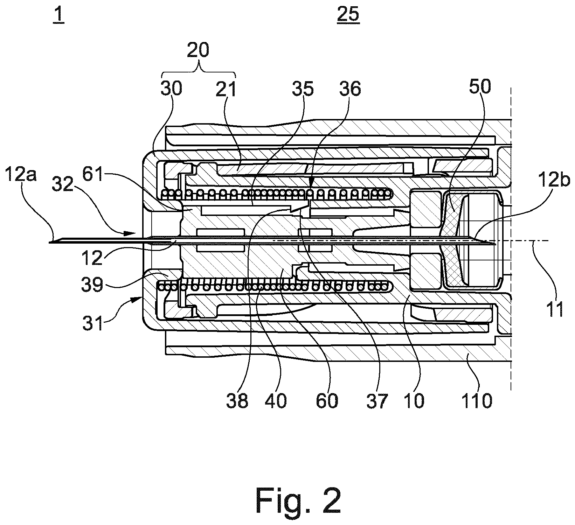

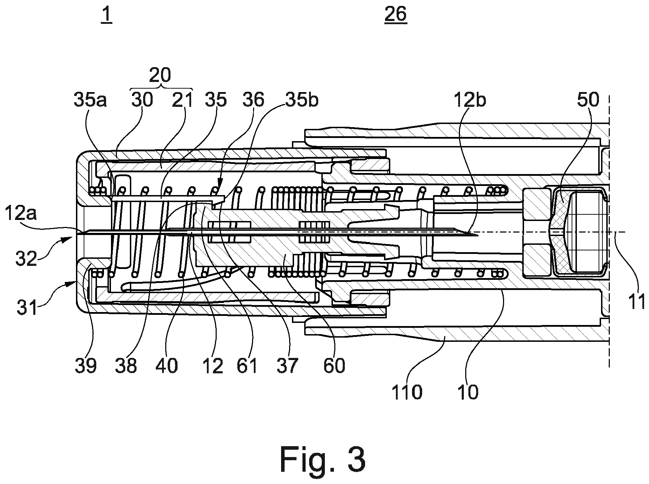

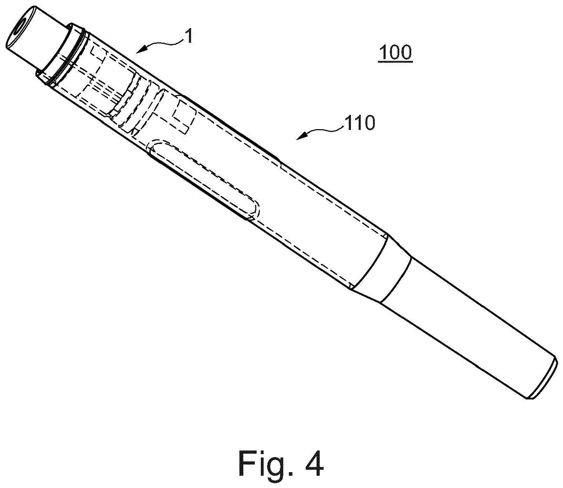

**Figs. 1 to 3** show the needle safety device 1 according to the present disclosure and / or parts thereof. The needle safety device 1 may be for a medical device, in particular for a medical injection device 100 (as can be gathered from Fig. 4). The needle safety device 1 comprises a hub portion 10 defining a longitudinal axis 11, wherein the hub portion 10 comprises a cannula 12 extending from the hub portion 10 substantially along the longitudinal axis 11. The needle safety device 1 further comprises a carrier element 60 arranged on the cannula 12, wherein the cannula 12 and the carrier element 60 are configured to be moved together along the longitudinal axis 11 relative to the hub portion 10. The needle safety device 1 also comprises a tubular arrangement 20 being movably arranged on the hub portion 10 to move along the longitudinal axis 11 relative to the hub portion 10, wherein the tubular arrangement 20 is configured to be moved between at least an initial position 24 (Figs. 1 and 3) and an injection position 25 (Fig. 2). In the injection position 25, a proximal end 12b of the cannula 12 is configured to protrude through a septum 50 of the medical injection device 100 such that the cannula 12 is in fluid communication with a liquid reservoir (not shown but the liquid reservoir could be arranged further to the right-hand side of Figs. 1 to 3 as seen along the longitudinal axis 11) of the medical device. The tubular arrangement 20 comprises a coupling means 35, configured to engage with the carrier element 60 such that the proximal end 12b of the cannula 12 is configured to be moved out of the septum 50 when the tubular arrangement 20 is moved from the injection position 25 (Fig. 2) to the initial position 24 (Figs. 1 and 3).

The movement between the initial position 24 (Figs. 1 and 3) and the injection position 25 may mean an axial movement in one example.

The cannula 12 may be attached to the carrier element 60. Various means for attachment are generally encompassed in the present disclosure whenever reference is made to two or more parts being attached. Such attachment means include one or more or all of the following non-exhaustive list: Fasteners (such as screws, bolts, nuts, rivets, clamps, staples), adhesives and glues (various types of adhesive substances may bond together when they dry or cure, a double-sided tape comprising adhesive on both sides of a tape), welding (melting portions of the parts at least partially and fusing them together), soldering (melting a low-temperature part for joining), mechanical connections (latches and catches, hooks and loops, zip ties), magnetic attachments, snap-fit connections (interlocking components that snap together). As understood by the skilled person, the way in which the two parts are attached to one another may depend on the materials involved and / or the specific intended purpose.

The longitudinal axis 11 of the hub portion 10 may be substantially parallel to the longitudinal axis of the coupling means 35. The longitudinal axis of the coupling means 35 may be offset to the longitudinal axis 11 of the hub portion 10. Further, the longitudinal axis 11 of the coupling means 35 may be offset to the longitudinal axis of the cannula 12. This facilitates that movement of the cannula 12 is not impaired by way of the coupling means 35.

The liquid reservoir may be a cartridge, a barrel or the like. In various examples, the liquid reservoir may be housed within a cylindrical glass or plastic container. The liquid reservoir may comprise a medication or a component thereof. It is noted that the liquid reservoir could alternatively or additionally be located further away from the septum 50. The fluid communication referred to herein merely serves the purpose that medication can be delivered, this may be independent on whether or not the liquid reservoir is located directly adjacent to the septum 50.

The septum 50 may prevent air, contaminants, and moisture from entering the liquid reservoir and affecting the stability and efficacy of the medication. Typically, when the medical device is activated, the cannula 12 pierces or punctures the septum 50 to access the medication. This piercing or puncture facilitates the medication to be drawn out of the liquid reservoir. The septum 50 may ensure that the medication remains sterile until the moment of administration.

The carrier element 60 may contact the hub portion 10. The carrier element 60 may particularly contact an inner peripheral surface of the hub portion 10 with an outer peripheral surface of the carrier element 60. Thereby, it may be possible to guide the carrier element 60 together with the cannula 12 within a part of the hub portion 10. Thus, the cannula 12 is movable relative to the hub portion 10, to realize a penetration of a liquid reservoir within the medical injection device.

The cannula 12 may be any kind of piercing means, such as a needle, or the like.

It is noted that the initial position 24 (Figs. 1 and 3) to which the tubular arrangement 20 is moved after being in the injection position 25 (Fig. 2) may in some examples additionally or alternatively be referred to as the final position 26 (as indicated in Fig. 3). This may mean that the needle safety device 1 may be capable of ensuring that the medical device may not be used a second time, although this is not precluded. In the final position 26, one or more parts of the needle safety device 1 may have a different arrangement as compared to the initial position 24 (for instance a rotation may have taken place of one or more parts of the needle safety device 1). However, the axial arrangement of the tubular arrangement 20 may be substantially similar in the initial position 24 and in the final position 26. In some examples, the final position 26 corresponds to the after-injection position described elsewhere herein.

The coupling means 35 may be configured to be displaced relative to the carrier element 60, preferably in a direction substantially perpendicular to the longitudinal axis 11, when the tubular arrangement 20 is moved from the initial position 24 to the injection position 25. In particular, the coupling means 35 may be configured to be in sliding engagement with the carrier element 60 when the tubular arrangement 20 is moved from the initial position 24 to the injection position 25. As can be imagined when looking at Fig. 1 and Fig. 2, the coupling means 35 may be resilient, such that it provides a restoring force in a direction substantially perpendicular to the longitudinal axis 11. Further, the coupling means 35 may have an elongated shape, wherein a longitudinal axis of the coupling means 35 is substantially parallel to the longitudinal axis 11 of the hub portion 10. In particular, as shown in the Figs. 1, 2, and 3, the couplings means 35 is a coupling element 35, in particular a coupling arm 35. In some examples, two or more coupling arms 35 may be encompassed.

The coupling means 35 may comprise a snap portion 36 configured to engage with the carrier element 60 when moved from the injection position 25 to the initial position 24 and the snap portion 36 may be configured to be in sliding engagement with the carrier element 60 when moved from the initial position 24 to the injection position 25. The snap portion 36 may have a tapered shape as seen along the longitudinal axis 11 from a distal end 35a of the coupling means 35 towards a proximal end 35b of the coupling means 35.

The snap portion 36 may have an inclined surface 37 configured to slide over the carrier element 60, in particular over a protrusion 61 of the carrier element 60, and an abutting surface 38 for engaging with the carrier element 60, in particular with the protrusion 61, wherein the inclined surface 37 is arranged in proximity of a proximal end 35b of the coupling means 35, wherein the abutting surface 38 is arranged further to a distal end 35a of the coupling means 35 with respect to the inclined surface 37.

The protrusion 61 of the carrier element 60 may be configured to engage with the coupling means 35 in particular with the snap portion 36 of the coupling means 35. The protrusion 61 may be something that extends from (likewise be termed to jut out) a surface or object, such as the carrier element 60. The protrusion 61 may be used to describe any part of an object or structure that juts out, sticks out, or extends beyond the surrounding surface or boundary. In one example, the protrusion 61 may be a three-dimensional extension or projection that extends outward from a surface or object. The protrusion 61 may be provided in various sizes. The size, shape, or the like of the protrusion 61 may vary depending on the indented purpose. Substantially all technically meaningful sizes and shapes may be encompassed in the present disclosure. The protrusion 61 facilitates a structural benefit and / or it can add more specific functionalities to the carrier element 60. For instance, the protrusion 61 may be specifically designed for the purpose of providing an engagement and a sliding engagement with the coupling means 35, in particular with the snap portion 36 of the regaining arm 35. The protrusion 61 may be integrally formed with the carrier element 60 or it may be attached to the carrier element 60 using any kind of attachment means described elsewhere herein.

The inclined surface 37 may define an angle with respect to the longitudinal axis 11 of the hub portion 10 of at least 10° and at most 80° as described elsewhere herein. The abutting surface 38 may be arranged substantially perpendicular to the longitudinal axis 11 of the hub portion 10. Further, also the protrusion 61 may extend in a direction substantially perpendicular to the longitudinal axis 11 of the hub portion 10. This allows for a combined advantageous effect in that the abutting surface 38 and the protrusion 61 can make a substantially flat contact.

The needle safety device 1 may further comprise a spring element 40 which biases the tubular arrangement 20 in a distal direction relative to the hub portion 10. The distal direction may be the direction from the injection position 25 to the initial position 24. It is noted that a movement of the tubular arrangement 20 from the injection position 25 to the initial position 24 may occur for instance, when pressure on a contact surface 31 (as described further below) of the tubular arrangement 20 is released. The spring element 40 may abut the hub portion 10 and the second tubular element 30. This configuration has proven to be advantageous in that it prevents the spring element 40 from adversely affecting the rotation of the first tubular element 21 (as described below). Further, the spring element 40 may be arranged at an outer periphery of the carrier element 60 and/or the coupling means 35.

As best seen in Fig. 2, the coupling means 35 may have a length along the longitudinal axis 11 such that a distal end 12a of the cannula 12 is arranged substantially in proximity of a plane defined by the contact surface 31 of the tubular arrangement 20 for establishing pressure contact with an injection area, when the coupling means 35 engages with the carrier element 60. This has the advantage that the coupling means 35 is long enough such that contact with the cannula 12 by a user is substantially prevented or at least a likelihood of such contact is reduced. For instance, since the distal end 12a of the cannula 12 does not extend out of the plane defined by the contact surface 31, a user may not easily get in contact therewith. It is understood that said engagement of the coupling means 35 with the carrier element 60 may occur, when the tubular arrangement 20 is moved from the injection position 25 to the initial position 24.

The tubular arrangement 20, in particular the second tubular element 30 (as described further below in greater detail) comprises a tubular projection 39 extending from the contact surface 31 towards a proximal end of the tubular arrangement 20, wherein a distal end 35a of the coupling means 35 is attached to the tubular projection 39 (as best seen in Fig. 3). In one example, the coupling means 35 may be integrally formed with the tubular arrangement 20, in particular with the tubular projection 39. A proximal end of the tubular projection 39 may contact the carrier element 60 in the injection position 25 (as best seen in Fig. 2). This may have the advantage that guiding the cannula 12 further into an injection area of a user can be prevented.

The contact surface 31 of the tubular arrangement 20, in particular of the second tubular element 30 serves the purpose to establish pressure contact with an injection area. The spring element 40 is configured such that the contact surface 31 is further spaced apart from the hub portion 10 along the longitudinal axis 11 than a distal end 12a of the cannula 12 when the contact surface is not in pressure contact with the injection area (Figs. 1 and 3).

In the following, some general features of the needle safety device 1 of the present disclosure are explained in greater detail, in particular with reference to Figs. 1 to 3.

The tubular arrangement 20 may comprise a first tubular element 21 and second tubular element 30. The first tubular element 21 may be movably arranged on the hub portion 10 to move along the longitudinal axis 11. The first tubular element 21 may comprise a guide track which slidably engages with a guide pin of the hub portion 10. The guide track may be configured such that the first tubular element 21 rotates relative to the hub portion 10 when the guide pin moves along the guide track, wherein the guide track may comprise a final location configured to retain the guide pin. The second tubular element 30 may be arranged on the first tubular element 21 such that the second tubular element 30 is movable along the longitudinal axis 11 together with the first tubular element 21. The first tubular element 21 may be at least partially enclosed by the second tubular element 30. The second tubular element 30 may comprise a contact surface 31 for establishing pressure contact with an injection area.

In addition to the advantages already set out in the present disclosure, the needle safety device 1 has the benefit that it may serve to prevent the reuse of a medical device, in particular a medical injection device 100, and to prevent accidental contact with the cannula 12. This may be achieved by way of the guide pin of the hub portion 10 described in the foregoing. Upon rotation of the first tubular element 21 relative to the hub portion 10, i.e., when the tubular arrangement 20 is moved from the initial position 24 to the injection position 25, the guide pin may substantially move along the guide track, wherein the guide track comprises a final location configured to retain the guide pin. This may mean that the guide pin is prevented from returning into other portions of the guide track.

**Fig. 4** shows a medical device, in particular a medical injection device 100 according to the present disclosure which comprises the needle safety device 1 as described above. The medical injection device 100 comprises a housing 110 which engages with the tubular arrangement 20, in particular with the second tubular element 30 of the tubular arrangement 20, such that the second tubular element 30 substantially does not rotate relative to the hub portion 10. Optionally, the hub portion 10 is affixed to the housing 110. Said fixing between the hub portion 10 and the housing 110 may be substantially permanently. However, the hub portion 10 may alternatively be releasably fixed to the housing 110. In this manner, user comfort and the applicability of the needle safety device 1 and / or the medical device may be increased. The medical injection device 100 can be an auto-injector or an injection pen or the like.

It is noted that any one or more of the embodiments described herein and / or examples may be combined with further aspects as described herein and details of the embodiments and / or examples may also be omitted, as will be understood by the skilled person. The scope of protection is determined by the claims and is not limited by the embodiments and / or examples disclosed in the above figures.

### List of reference signs

- 1: needle safety device
- 10: hub portion
- 11: longitudinal axis
- 12: cannula
- 12a: distal end of the cannula
- 12b: proximal end of the cannula
- 20: tubular arrangement
- 21: first tubular element
- 24: initial position
- 25: injection position
- 26: final position
- 30: second tubular element
- 31: contact surface of the tubular arrangement / of the second tubular element
- 32: opening in the contact surface
- 35: coupling means
- 35a: distal end of the coupling means
- 35b: proximal end of the coupling means
- 36: snap portion
- 37: inclined surface
- 38: abutting surface
- 39: tubular projection
- 50: septum
- 60: carrier element
- 61: protrusion
- 40: spring element
- 100: medical injection device
- 110: housing

## Claims

1. A needle safety device (1) for a medical device, in particular for a medical injection device (100), the needle safety device (1) comprising:
a hub portion (10) defining a longitudinal axis (11), wherein the hub portion (10) comprises a cannula (12) extending from the hub portion (10) substantially along the longitudinal axis (11) and a carrier element (60) arranged on the cannula (12), wherein the cannula (12) and the carrier element (60) are configured to be moved together along the longitudinal axis (11) relative to the hub portion (10);
a tubular arrangement (20) being movably arranged on the hub portion (10) to move along the longitudinal axis (11) relative to the hub portion (10), wherein the tubular arrangement (20) is configured to be moved between at least an initial position (24) and an injection position (25),
wherein in the injection position (25), a proximal end (12b) of the cannula (12) is configured to protrude through a septum (50) of the medical device such that the cannula (12) is in fluid communication with a liquid reservoir of the medical device,
wherein the tubular arrangement (20) comprises coupling means (35), configured to engage with the carrier element (60) such that the proximal end (12b) of the cannula (12) is configured to be moved out of the septum (50) when the tubular arrangement (20) is moved from the injection position (25) to the initial position (24).

2. The needle safety device (1) according to the preceding claim, wherein the coupling means (35) is configured to be displaced relative to the carrier element (60), preferably in a direction substantially perpendicular to the longitudinal axis (11), when the tubular arrangement (20) is moved from the initial position (24) to the injection position (25).

3. The needle safety device (1) according to any one of the preceding claims,
wherein the coupling means (35) is configured to be in sliding engagement with the carrier element (60) when the tubular arrangement (20) is moved from the initial position (24) to the injection position (25).

4. The needle safety device (1) according to any one of the preceding claims,
wherein the coupling means (35) is resilient, such that it provides a restoring force in a direction substantially perpendicular to the longitudinal axis (11).

5. The needle safety device (1) according to any one of the preceding claims,
wherein the coupling means (35) has an elongated shape, wherein a longitudinal axis of the coupling means (35) is substantially parallel to the longitudinal axis (11) of the hub portion (10).

6. The needle safety device (1) according to any one of the preceding claims,
wherein the coupling means (35) comprises a snap portion (36) configured to engage with the carrier element (60) when moved from the injection position (25) to the initial position (24) and configured to be in sliding engagement with the carrier element (60) when moved from the initial position (24) to the injection position (25).

7. The needle safety device (1) according to claim 6, wherein the snap portion (36) has a tapered shape as seen along the longitudinal axis (11) from a distal end (35a) of the coupling means (35) towards a proximal end (35b) of the coupling means (35).

8. The needle safety device (1) according to any one of claims 6 or 7, wherein the snap portion (36) has an inclined surface (37) configured to slide over the carrier element (60), in particular over a protrusion (61) of the carrier element (60), and an abutting surface (38) for engaging with the carrier element (60), in particular with a protrusion (61) of the carrier element (60), wherein the inclined surface (37) is arranged in proximity of a proximal end of the coupling means (35), wherein the abutting surface (38) is arranged further to a distal end of the coupling means (35) with respect to the inclined surface (37).

9. The needle safety device (1) according to claim 8, wherein the inclined surface (37) defines an angle with respect to the longitudinal axis (11) of the hub portion of at least 10°, preferably at least 20°, more preferably at least 30°, more preferably at least 40°, more preferably at least 45°, and/or of at most 80°, preferably at most 70°, more preferably at most 60°, more preferably at most 50°, most preferably at most 45°.

10. The needle safety device (1) according to any one of claims 8 or 9, wherein the abutting surface (38) is arranged substantially perpendicular to the longitudinal axis (11) of the hub portion (10).

11. The needle safety device (1) according to any one of the preceding claims,
wherein the carrier element (60) comprises a protrusion (61) for engaging with the coupling means (35) in particular with the snap portion (36) of the coupling means (35).

12. The needle safety device (1) according to claim 11, wherein the protrusion (61) extends in a direction substantially perpendicular to the longitudinal axis (11).

13. The needle safety device (1) according to any one of the preceding claims, further comprising a spring element (40) which biases the tubular arrangement (20) in a distal direction relative to the hub portion (10).

14. A medical device, in particular a medical injection device (100), comprising a needle safety device (1) according to any one of the preceding claims.

15. The medical device of the preceding claim, wherein the medical injection device (100) is an auto-injector or an injection pen.
